# DEMANDE DE BREVET EUROPEEN

(11) **EP 2 014 320 A1**
(43) Date de publication de la demande: **14.01.2009**
(21) Numéro de dépôt: 07360031.4
(22) Date de dépôt: 10.07.2007
(51) Int. Cl.: A61L 31/04, A61L 31/14

(54) **Pansement durcissable par UV**

(71) Demandeur: Lesca, Christophe, 75016 Paris (FR)
(72) Inventeur: Lesca, Christophe, 75016 Paris (FR)
(74) Mandataire: Littolff, Denis

(57) **Abrégé**

Pansement associant une membrane souple à une substance chimique disponible dans un état susceptible d'imprégner la membrane et dont le durcissement est contrôlé. Le durcissement de ladite substance s'effectue par rayonnement ultraviolet.

## Description

La présente invention concerne un pansement utilisable en chirurgie et associant notamment une toile stérilisable à une substance chimique dont le durcissement est contrôlé.

L'association de telles toiles et de colles durcissantes, par exemple au contact de l'air, est déjà connue. Toutefois, dans ce cas, il n'y a guère de maîtrise du facteur temps : dès lors que la toile est imprégnée par la colle autodurcissante, le chirurgien doit sans perdre de temps la placer sur le lieu de son application, avant qu'elle ne durcisse et ne rende l'opération impossible.

Le but de la présente invention est de ne plus dépendre du facteur temps, ou à tout le moins de mieux maîtriser cette contrainte temporelle au cours du maniement du pansement. L'emploi d'une colle cyanoacrylate classique, qui polymérise dans sa consistance visqueuse en une minute à une minute trente ne permet pas d'utiliser des pansements pour toutes leurs applications potentielles du fait de la fenêtre temporelle restreinte qu'ils offrent.

Par ailleurs, un autre objectif de l'invention est de s'adapter au mieux à certaines contraintes liées aux sites d'utilisation de ces pansements, afin d'assurer une efficacité maximale du durcissement quel que soit le type d'application.

A ces effets, le pansement de l'invention, associant comme on l'a dit une membrane souple à une substance chimique disponible dans un état susceptible d'imprégner la membrane, et dont le durcissement est contrôlé, se caractérise notamment en ce que ledit durcissement s'effectue par polymérisation par rayonnement ultraviolet.

L'avantage principal de ce traitement est le suivant : l'emploi d'une telle substance permet de s'affranchir de toute contrainte temporelle, l'utilisateur étant maître en toutes circonstances du facteur temps en décidant du moment de la polymérisation.

Ladite substance peut alors être une colle acrylique UV.

Alternativement, le durcissement peut s'effectuer par polymérisation selon une double stimulation :
- par la vapeur d'eau ;
- par rayonnement ultraviolet.

L'intérêt de cette double stimulation est le suivant : l'unique exposition à des rayonnements ultraviolets présente l'inconvénient que ceux-ci n'accèdent pas forcément dans tous les cas à la partie profonde du pansement, au contact des muqueuses recouvertes, et la polymérisation de la colle n'est donc pas assurée à ces emplacements.

L'utilisation de la composante vapeur d'eau permet en revanche de faire polymériser la colle au contact de la muqueuse, grâce à la présence de sang. Les rayons UV, quant à eux, permettent de maîtriser le durcissement de la partie superficielle du pansement en fin d'intervention, afin d'étanchéifier l'ensemble.

Les deux actions se combinent et renforcent l'efficacité du pansement.

Le problème de l'action des rayons ultraviolets en profondeur au niveau des surfaces recouvertes par les pansements se pose avec d'autant plus d'acuité que la membrane, par exemple une toile de gaze de cellulose, est épaisse. Dans ce cas, il est d'ailleurs également tout à fait envisageable, selon un protocole d'utilisation possible, de passer la face profonde du pansement sous la source de rayonnement ultraviolet pendant une fraction de temps, afin de démarrer la polymérisation sur cette face. Une telle étape aurait pour intérêt supplémentaire de commencer à rigidifier le pansement, de manière à pouvoir le conformer plus facilement sur le site à recouvrir.

La polymérisation se termine ensuite, à ce niveau, du fait de l'humidité notamment présentée par le sang.

Selon une possibilité, la substance en question peut être une colle de type cyanoacrylate. Alternativement, il peut s'agir d'une substance basée sur du silicone polymérisable, soit uniquement par exposition à un rayonnement UV, soit par la double stimulation précitée. Cette substance peut comprendre au moins un activateur sensible à une longueur d'onde, et de préférence plusieurs. Ainsi, selon un exemple possible, elle peut comprendre trois activateurs répondant à trois longueurs d'onde distinctes, ce qui facilite son emploi et permet de l'utiliser avec des sources de rayonnement ultraviolet classiques, faciles à acquérir et disponibles le cas échéant pour d'autres usages.

L'un des points fondamentaux liés à l'utilisation du pansement selon l'invention réside dans la possibilité de son emploi de manière extemporanée. Ainsi, la membrane et la substance durcissante peuvent à cet effet être emballées dans un emballage constitué de deux logements distincts séparés par une cloison permettant le passage de ladite substance vers le logement de la membrane lorsque la substance est refoulée de son propre logement.

L'entrée de la colle, ou plus généralement de la substance durcissante, dans la zone contenant la membrane permet de réaliser une imprégnation suffisante de cette dernière avant ouverture du reste de l'emballage pour utilisation immédiate du pansement. De préférence, le logement de la substance durcissante est délimité par des parois opaques à la lumière, afin de la prémunir contre tout risque de polymérisation intempestive.

Plus précisément, l'emballage peut consister en deux blisters en matière souple réunis par une cloison laissant passer la substance durcissante d'un blister à l'autre par déchirure ou perméabilité, le refoulement de ladite substance de l'un vers l'autre s'effectuant alors par pression sur le blister de la substance durcissante.

L'utilisation d'une matière souple pour les blisters permet ensuite l'imprégnation de la membrane par des manipulations de son blister.

L'emploi d'un tel double blister est intéressant à plusieurs titres :
- le problème souvent rencontré des dates de péremption de produits reconstitués serait très facilement résolu par le fait que la date la plus proche serait en l'occurrence la date déterminante ;
- pour le moment, on ne connaît pas les effets dans le temps de l'interaction de ce type de colle avec une membrane de cellulose oxydée. La préparation ayant eu lieu de manière extemporanée, ce problème ne se pose plus ;
- enfin, il est beaucoup plus difficile de conditionner une membrane avec une colle cyanoacrylate sous vide, en dehors de toute vapeur d'eau pour éviter tout début de polymérisation. En revanche, il est relativement facile de conditionner la membrane sèche dans un vide suffisant.

Alternativement, l'emballage peut être un flacon souple muni d'un embout, permettant de déposer la colle directement sur la toile, soit dans son blister, soit après découpage.

L'invention concerne également un procédé de préparation d'un pansement obéissant aux conditions précitées, caractérisé en ce qu'il consiste à :
- sortir la membrane du logement de son emballage ;
- la découper aux dimensions requises par l'application qui va en être faite ;
- remettre la membrane dans son logement ;
- refouler la substance durcissante de son logement de sorte qu'elle pénètre dans le logement de la membrane ; et
- manipuler le logement de la membrane pour réaliser l'imprégnation.

Il est plus difficile de découper une toile déjà encollée, du fait du risque d'encollage des instruments chirurgicaux lors de la manipulation. Au lieu de proposer le découpage des pansements à l'aide d'instruments à usage unique, qui complique la manoeuvre et le conditionnement du produit, et n'est en outre guère favorable en termes économiques, on procède à la découpe avant imprégnation. La mise en place, au niveau de l'application qui doit être faite du pansement, peut ensuite se faire en amenant le pansement sur le site à protéger avec les doigts, ou avec des pinces chirurgicales, et en le maintenant appliqué par des mouvements digitaux jusqu'au début de la polymérisation. La suture du pansement intervient alors, puis l'exposition à des rayonnements ultraviolets permet de faire durcir l'ensemble. En cas de persistance d'une petite suffusion hémorragique, un complément de colle peut être amené directement sur le pansement par exemple à l'aide du flacon et de l'embout, éventuellement étalé avec les doigts, avant une polymérisation spontanée ou aux ultraviolets

Parmi les applications envisagées, il est possible d'unir deux vaisseaux sanguins dont les berges de section sont maintenues au contact l'une de l'autre à l'aide d'une membrane préencollée selon l'invention. Un point de suture, voire un deuxième, pourrait être positionné pour bien appliquer les deux berges l'une en face de l'autre. Le pansement encollé, ceinturant l'anastomose, serait ensuite appliqué de manière à assurer l'étanchéité du montage.

Il est également possible d'utiliser une membrane et une substance durcissante selon l'invention pour une durée limitée et dans l'application suivante : la membrane est constituée d'une toile incolore transparente destinée à permettre une traction cutanée ponctuelle dans le temps. Ainsi, il pourrait être possible de coller une membrane incolore invisible non résorbable sur la peau de la partie postérieure du cou, au ras de la ligne d'implantation du cuir chevelu, en arrière et en dessous des oreilles.

Des petites languettes de cette membrane, éventuellement protégées de l'encollement au départ, pourraient dépasser vers l'arrière dans le cuir chevelu et être reliées l'une à l'autre, en arrière du cuir chevelu, par un fil incolore afin de tracter vers l'arrière l'ensemble de la peau du cou. Ce ou ces fils viendraient alors se cliper sur les languettes.

Un tel dispositif pourrait satisfaire, le temps d'une soirée, une femme refusant toute chirurgie esthétique cervicale. Dans le domaine de la création d'une tension superficielle cutanée, une autre application peut être envisagée

Actuellement, il existe des pansements encollés sur une face (pas par de la colle cyanoacrylate) permettant de ponter des petites plaies pouvant remplacer les points de suture du plan cutané : ce sont les pansements connus sous le nom commercial stéri-strips. On peut ainsi imaginer des petites languettes de toile - en l'espèce non résorbables - noyées dans de la colle type cyanoacrylate et qui, retirées de leur blister, pourraient ponter une plaie à la manière desdits pansements stéristrips. L'inconvénient est qu'il faudrait garder les berges d'une plaie au contact l'une de l'autre jusqu'à polymérisation. L'avantage est que le collage serait beaucoup plus efficace et beaucoup plus durable dans le temps.

Selon un autre exemple, l'invention pourrait être utilisée pour traiter des fractures à l'aide d'une toile encollée. Cette toile est alors positionnée sur les berges osseuses de la fracture une fois celle-ci réduite. La membrane, dans ce cas, doit être prévue relativement épaisse pour devenir rigide une fois la colle polymérisée. La membrane rigidifiée collée à l'os a alors les propriétés d'une plaque métallique d'ostéosynthèse. Elle pourrait d'ailleurs également être solidarisée à l'os par des vis.

Ce dispositif, non résorbable par l'organisme, doit en principe être retiré par la suite, une fois la consolidation terminée.

Bien entendu, l'invention permet d'autres applications, qu'il n'est pas possible de décrire de manière exhaustive.

## Revendications

1. Pansement associant une membrane souple à une substance chimique disponible dans un état susceptible d'imprégner la membrane et dont le durcissement est contrôlé, **caractérisé en ce que** le durcissement de ladite substance s'effectue par rayonnement ultraviolet.

2. Pansement selon la revendication précédente **caractérisé en ce que** ladite substance est une colle acrylique UV.

3. Pansement selon la revendication précédente **caractérisé en ce que** le durcissement de ladite substance s'effectue par polymérisation selon une double stimulation :
- par la vapeur d'eau ;
- par rayonnement ultraviolet.

4. Pansement selon la revendication précédente, **caractérisé en ce que** ladite substance est une colle de type cyanoacrylate.

5. Pansement selon l'une des revendications 1 et 3 **caractérisé en ce que** la substance est basée sur du silicone polymérisable.

6. Pansement selon l'une des revendications précédentes, **caractérisé en ce que** la substance comprend au moins un activateur sensible à une longueur d'onde.

7. Pansement selon la revendication précédente, **caractérisé en ce que** la substance comprend trois activateurs répondant à trois longueurs d'ondes distinctes.

8. Pansement selon l'une quelconque des revendications précédentes, **caractérisé en ce que** la membrane et la substance durcissante sont emballées dans un emballage constitué de deux logements distincts séparés par une cloison permettant le passage de ladite substance vers le logement de la membrane lorsqu'elle est refoulée de son propre logement.

9. Pansement selon la revendication précédente, **caractérisé en ce que** le logement de la substance durcissante est délimité par des parois opaques à la lumière.

10. Pansement selon l'une des revendications 8 et 9, **caractérisé en ce que** l'emballage consiste en deux blisters en matière souple réunis par une cloison laissant passer la substance durcissante d'un blister à l'autre par déchirure ou perméabilité, le refoulement de ladite substance de l'un vers l'autre s'effectuant par pression sur le blister de la substance durcissante.

11. Pansement selon la revendication précédente, **caractérisé en ce que** l'imprégnation de la membrane s'effectue par manipulation de son blister.

12. Pansement selon l'une quelconque des revendications précédentes, **caractérisé en ce que** la membrane est en toile de gaze de cellulose.

13. Procédé de préparation d'un pansement selon les revendications 8 à 12, **caractérisé en ce qu'**il consiste à :
- sortir la membrane du logement de son emballage ;
- la découper aux dimensions requises par l'application qui va en être faite ;
- remettre la membrane dans son logement ;
- refouler la substance durcissante de son logement de sorte qu'elle pénètre dans le logement de la membrane ; et
- manipuler le logement de la membrane pour réaliser l'imprégnation.
